(19) Europäisches Patentamt
European Patent Office
Office européen des brevets

(11) **EP 3 701 933 B1**

(12) **EUROPEAN PATENT SPECIFICATION**

(45) Date of publication and mention of the grant of the patent:
**21.01.2026 Bulletin 2026/04**

(21) Application number: **18869814.6**

(22) Date of filing: **24.10.2018**

(51) International Patent Classification (IPC):
***A61K 8/44*** *(2006.01)* ***A61K 8/63*** *(2006.01)*
***A61Q 1/10*** *(2006.01)* ***A61Q 19/00*** *(2006.01)*
***A61K 8/31*** *(2006.01)* ***A61K 8/58*** *(2006.01)*
***A61K 8/06*** *(2006.01)* ***A61K 8/25*** *(2006.01)*
***A61K 8/34*** *(2006.01)* ***A61K 8/891*** *(2006.01)*
***A61K 8/86*** *(2006.01)*

(52) Cooperative Patent Classification (CPC):
**A61K 8/86; A61K 8/062; A61K 8/25; A61K 8/345; A61K 8/891; A61Q 1/10; A61Q 19/00**

(86) International application number:
**PCT/JP2018/039432**

(87) International publication number:
**WO 2019/082911 (02.05.2019 Gazette 2019/18)**

(54) **OIL-IN-WATER EMULSION COMPOSITION AND COSMETIC PREPARATION**

ÖL-IN-WASSER-EMULSIONSZUSAMMENSETZUNG UND KOSMETISCHES PRÄPARAT

COMPOSITION D'ÉMULSION D'HUILE DANS L'EAU ET PRÉPARATION COSMÉTIQUE

(84) Designated Contracting States:
**DE FR GB**

(30) Priority: **27.10.2017 JP 2017207852**

(43) Date of publication of application:
**02.09.2020 Bulletin 2020/36**

(73) Proprietor: **Shin-Etsu Chemical Co., Ltd.**
**Tokyo 100-0004 (JP)**

(72) Inventors:
- **TAKADA Yuko**
**Annaka-shi**
**Gunma 379-0224 (JP)**
- **UBUKATA Saori**
**Annaka-shi**
**Gunma 379-0224 (JP)**
- **TAWATA Hanako**
**Annaka-shi**
**Gunma 379-0224 (JP)**
- **TAKEWAKI Kazuyuki**
**Annaka-shi**
**Gunma 379-0224 (JP)**

(74) Representative: **Mewburn Ellis LLP**
**Aurora Building**
**Counterslip**
**Bristol BS1 6BX (GB)**

(56) References cited:
**EP-A1- 3 558 460**
**WO-A1-01/15658**
**FR-A1- 2 968 973**
**JP-A- 2004 346 046**
**JP-A- 2004 346 046**
**JP-A- 2007 284 376**
**US-A1- 2004 241 126**
**US-A1- 2007 148 115**
**US-A1- 2015 196 467**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 701 933 B1

## Description

### TECHNICAL FIELD

The present invention relates to silicone resin-containing oil-in-water emulsion compositions and cosmetic preparations.

### BACKGROUND ART

Resins which are water resistant and oil resistant so as to impart good properties to cosmetic preparations and which prevent makeup from coming off have been developed in the field of makeup cosmetics, including eye makeup such as mascara and skin care products. Silicone oils and silicone resins having water repellency are commonly used as such resins, the important qualities of which are to keep makeup from wearing off and give it excellent longevity. Examples of water-repelling silicone oils and silicone resins used in cosmetic preparations include crosslinked silicones obtained by the addition polymerization of a silicone resin or an organohydrogenpolysiloxane with a vinyl group-containing organopolysiloxane, and acrylic-silicone graft copolymers (Patent Document 1: JP No. 2796856; Patent Document 2: JP No. 2704730; Patent Document 3: JP-A 2017-66092). Of these, silicone resins, given the ease of adjusting the film hardness by varying the ratio of crosslinked units and the degree of polymerization, are employed in many cosmetic preparations by selectively using the structure according to the intended use of the cosmetic preparation.

In recent years, owing to concerns over environmental impact and irritation of the skin, not only water-in-oil cosmetic preparations, but also silicone resin-containing oil-in-water emulsion compositions, are commonly used in makeup cosmetics (Patent Document 4: JP No. 4846423). Because silicone resins are solids or highly viscous liquids and difficult to emulsify in this form, the silicone resin is generally dissolved in a hydrocarbon solvent such as isododecane or a low-viscosity silicone oil, and then mixed with a surfactant and emulsified. When intended for a cosmetic preparation, the silicone resin is dissolved by using an amount of solvent that is equal to or greater than the amount of silicone resin. The solvent sometimes interferes with the properties of the silicone resin or causes irritation of the skin (Patent Document 3: JP-A 2017-66092; Patent Document 4: JP No. 4846423; Patent Document 5: JP No. 3220953). Hence, there exists a desire for oil-in-water emulsion compositions in which the proportion of hydrocarbon solvent such as isododecane, or of low-viscosity silicone oil, with respect to the silicone resin is low. However, when the proportion of hydrocarbon solvent such as isododecane, or of low-viscosity silicone oil, with respect to the silicone resin is low, cracks may arise in the film, solids may settle out, or crawling may occur. In addition, it may be difficult to achieve a small particle size in emulsion particles of the oil-in-water emulsion composition, and so concentration separation may occur over time. Silicone resin emulsion compositions in which, based on the silicone resin, from 5 to 25 wt% of a specific water-miscible organic solvent has been included and emulsified have hitherto been described (Patent Document 6: JP No. 5163888; Patent Document 7: JP-A 2008-138059). Yet, these solvents cannot be used in cosmetic preparations, and so there has existed a desire for silicone resin emulsion compositions that use a small amount of solvent suitable for use in cosmetic preparations.

Polyoxyethylene alkyl ethers such as polyoxyethylene lauryl ether and polyoxyethylene decyl ether are commonly used as surfactants for emulsifying silicone resins (Patent Document 5: JP No. 3220953; Patent Document 6: JP No. 5163888; Patent Document 7: JP-A 2008-138059), but these surfactants irritate the skin and so there is concern over their use. There is thus a desire for the use of surfactants which cause little irritation of the skin and are gentle even when taken up by the body. Polyglycerol fatty acid esters are relatively non-irritating and moreover recognized as food additives, but they have a weak emulsifying ability. Also, oil-in-water emulsion compositions obtained by emulsification with polyglycerol fatty acid esters may undergo concentration separation over time.

Hence, there exists a desire for silicone resin oil-in-water emulsion compositions which, when included in makeup cosmetics such as mascara and other eye makeup and skin care products, further improve the longevity (long-lasting properties) of cosmetic preparations, minimize cracking of the cosmetic preparation, the settling out of solids and crawling, and moreover are mild to the skin, gentle even when taken up by the body and have a good shelf stability.

Patent Document 8 discloses a cosmetic composition comprising, in a physiologically acceptable medium, at least 5% by weight of water; at least alkylcellulose, the alkyl residue of which comprises between 1 and 6 carbon atoms; at least one first hydrocarbon-based non-volatile oil; at least one second non-volatile oil selected from silicone oils and/or fluoro oils or hydrocarbon-based oils other than the first oil; and at least one stabilizer selected from surfactants and/or hydrophilic gelling agents.

### PRIOR ART DOCUMENTS

### PATENT DOCUMENTS

Patent Document 1: JP No. 2796856

Patent Document 2: JP No. 2704730

Patent Document 3: JP-A 2017-66092

Patent Document 4: JP No. 4846423

Patent Document 5: JP No. 3220953

Patent Document 6: JP No. 5163888

Patent Document 7: JP-A 2008-138059

Patent Document 8: FR 2968973 A1

## SUMMARY OF THE INVENTION

### TECHNICAL PROBLEM

In the light of the above circumstances, one object of this invention is to provide silicone resin-containing oil-in-water emulsion compositions which, when included in makeup cosmetics such as mascara and other eye makeup and skin care products, further improve the longevity of the cosmetic preparation, discourage crawling and cracking on the skin and the settling out of solids, and moreover are mild to the skin, gentle even when taken up by the body and have a good shelf stability. Another object is to provide cosmetic preparations formulated with such compositions.

### SOLUTION TO THE PROBLEM

The inventors have conducted extensive investigations in order to achieve these objects and discovered as a result that the above problems can be resolved by an oil-in-water emulsion composition obtained by emulsifying (A) a silicone resin composition which is composed of (A-1) a specific silicone resin and (A-2) a volatile oil that are mixed in a specific ratio and which has a kinematic viscosity at 25°C of from 100 to 4,000 $mm^2/s$ with (B) a polyoxyethylene sorbitan fatty acid ester having an average number of added moles of ethylene oxide (EO) of at least 10 and a hydrophilic-lipophilic balance (HLB) of at least 11. This discovery ultimately led to the present invention. More specifically, in this invention, the ratio between the silicone resin (A-1) and the volatile oil (A-2) is from 70/30 to 92/8; hence, the proportion of volatile oil (A-2) is low compared with conventional products made of a silicone resin dissolved in a volatile oil, and so the properties of the silicone resin (A-1) tend to remain intact and there is little irritation of the skin by the volatile oil. Moreover, by selecting a silicone resin such as to set the kinematic viscosity of the silicone resin composition (A) in the range of 100 to 4,000 $mm^2/s$, emulsification is possible with an ordinary emulsifier such as a homogenizing mixer or a homogenizing disperser; that is, without the use of a high-pressure homogenizer. As a result, emulsion particles in the oil-in-water emulsion composition thus produced are small in size, making the composition stable because problems such as concentration separation do not arise over time. Also, the oil-in-water emulsion composition of the invention uses a polyoxyethylene sorbitan fatty acid ester that is recognized as a food additive, and so it is mild to the skin and is gentle even when taken up within the body.

Accordingly, the invention provides the oil-in-water emulsion composition as defined in the claims.

### ADVANTAGEOUS EFFECTS OF THE INVENTION

The invention makes it possible to provide silicone resin-containing oil-in-water emulsion compositions which, when included in makeup cosmetics such as mascara and other eye makeup and skin care products, further improve the longevity of the cosmetic preparations, minimize crawling, cracking and the settling out of solids on the skin, and moreover are mild to the skin, gentle even when taken up by the body and have a good shelf stability. The invention also makes it possible to provide cosmetic preparations containing such compositions.

### DESCRIPTION OF EMBODIMENTS

The invention is described in detail below.

COMPONENT (A)

Component (A) is a silicone resin composition which is a mixture consisting of (A-1) a silicone resin having [$R_3SiO_{0.5}$] units and [$SiO_2$] units in a molar ratio, expressed as [$R_3SiO_{0.5}$]/[$SiO_2$], of from 0.7 to 1.4 (wherein each R is independently a monovalent organic group of 1 to 10 carbon atoms) and (A-2) a volatile oil having a boiling point at normal pressure of 260°C or less, in a weight ratio, expressed as (A-1) /(A-2), of from 70/30 to 92/8, and which has a kinematic viscosity at 25°C of from 100 to 4,000 $mm^2/s$.

Component (A-1) is a silicone resin having [$R_3SiO_{0.5}$] units and [$SiO_2$] units in a molar ratio, expressed as [$R_3SiO_{0.5}$]/[$SiO_2$], of from 0.7 to 1.4. One such silicone resin may be used alone or two or more may be suitably combined and used together. In the formula, each R is a like or unlike monovalent organic group of 1 to 10 carbon atoms. Specific examples include alkyl groups such as methyl, ethyl, propyl, butyl, pentyl, hexyl, heptyl, octyl, nonyl, decyl, cyclopentyl, cyclohexyl and cycloheptyl groups; aryl groups such as the phenyl group; and alkenyl groups such as vinyl and allyl groups. Of these, methyl and phenyl groups are preferred; methyl groups are especially preferred from the standpoint of versatility.

The molar ratio expressed as [$R_3SiO_{0.5}$]/[$SiO_2$] is from 0.7 to 1.4, and preferably from 1.1 to 1.4. At less than 0.7, the film is hard and cracks may arise in the film or solids may settle out. At more than 1.4, the film is too soft and, when used as a cosmetic film, does not exhibit a long-lasting effect. Aside from [$R_3SiO_{0.5}$] and [$SiO_2$] units, the silicone resin may also have [$R_2SiO$] units or [$RSiO_{1.5}$] units. The combined amount of [$R_3SiO_{0.5}$] units and [$SiO_2$] units represent a proportion of all the siloxane units that is preferably from 30 to 100 mol%, more preferably from 50 to 100 mol%, and even more preferably from 80 to 100 mol%. The [$R_2SiO$] unit content may be set to from 0 to 70 mol%, and preferably from 0 to 50 mol%, of all the siloxane units. At more than 70%, the film may be too soft and, when the oil-in-water emulsion composition is included in a cosmetic preparation, may not exhibit a long-lasting effect. The [$RSiO_{1.5}$] unit content may be set to from 0 to 50 mol%, and preferably from 0 to 20 mol%, of all the siloxane units. At more than 50%, stickiness may arise when the oil-in-water emulsion composition is included in a cosmetic preparation.

Also, component (A-1) may include SiOH groups and $SiOR^1$ groups. $R^1$ is exemplified by alkyl groups such as methyl, ethyl, propyl, butyl, pentyl, hexyl, heptyl, octyl, nonyl, decyl, cyclopentyl, cyclohexyl and cycloheptyl groups, and aryl groups such as the phenyl group. The SiOH and $SiOR^1$ groups may be included as, for example, [$HOSiO_{1.5}$] groups and [$R^1OSiO_{1.5}$] groups. Although the content of SiOH groups is not particularly specified, when such groups are included, the content is preferably from 0.015 to 0.3 mol/100 g. At more than 0.3 mol/100 g, the SiOH groups on the silicone resin within the oil-in-water emulsion composition may react with each other or the SiOH groups and $SiOR^1$ groups on the silicone resin may mutually react, which may change the silicone resin degree of polymerization. Although the content of $SiOR^1$ groups is not particularly specified, it is preferably from 0 to 0.5 mol/100 g. Within the oil-in-water emulsion composition, the $SiOR^1$ groups on the silicone resin (A-1) gradually hydrolyse, forming an alcohol. However, when the $SiOR^1$ group content is 0.5 mol/100 g or more, the amount of alcohol that forms due to hydrolysis increases, and this alcohol may interfere with the properties of the cosmetic preparation.

Component (A-1) has a weight-average molecular weight of preferably from 800 to 20,000, more preferably from 1,000 to 6,000, and even more preferably from 1,000 to 3,000. When the weight-average molecular weight is less than 800, it may be impossible to exhibit a long-lasting effect when the oil-in-water emulsion composition is included in a cosmetic preparation. On the other hand, when it exceeds 20,000, the film may be hard and cracks may arise therein, solids may settle out, or crawling may occur. Weight average molecular weights in this invention are polystyrene-equivalent weight-average molecular weights obtained by gel permeation chromatography (GPC). Component (A-1) is preferably a liquid silicone resin having an absolute viscosity at 36°C --the temperature of the human bodyof at least 5,000 mPa·s. Specifically, the silicone resin is such that, when placed in a container at 36°C and the container is tilted 90°, the shape of the silicone resin changes within one hour. By using a liquid silicone resin having fluidity at 36°C, coating unevenness and cracks do not arise even when applied onto human skin, and a film in which solids do not settle out can be formed. The absolute viscosity of component (A-1) at 36°C is preferably at least 5,000 mPa·s, and more preferably at least 8,000 mPa·s. If the silicone resin (A-1) has an absolute viscosity at 36°C of less than 5,000 mPa·s, a long-lasting effect may not be exhibited when the oil-in-water emulsion composition is included in a cosmetic preparation. The absolute viscosity of component (A-1) is a value measured with a BM-type rotational viscometer.

Component (A-1) can be produced by a known method, such as by hydrolysis and condensation reactions on chlorosilane and an alkoxysilane. For example, a silicone resin that is an MQ resin can be obtained by adding methanesulfonic acid to an isopropanol solution of hexamethylsiloxane and ethyl silicate or ethyl polysilicate, and subsequently carrying out hydrolysis and condensation reactions by the dropwise addition of water and 8 hours of refluxing at about 80°C. An alkaline aqueous solution is added to the resulting resin solution to neutralize the acid, following which a hydrocarbon solvent or a low-viscosity silicone oil is added and heating is carried out under reduced pressure, driving off the alcohol component and thereby giving a solution of the MQ resin in the hydrocarbon solvent or the low-viscosity silicone resin oil.

Component (A-2)

The volatile oil is one that can be used in cosmetic products and has a boiling point at normal pressure/25°C of 260°C or less. Such a volatile oil may be used singly or two or more may be used in suitable combination. Exemplary volatile oils include silicone oils, hydrocarbon oils and ester oils. Of these, from the standpoint of versatility, silicone oils and hydrocarbon oils are preferred.

The silicone oil used is one that has a kinematic viscosity at 25°C of 2 $mm^2/s$ or less. This viscosity is the kinematic viscosity, which is a value measured at 25°C with an Ostwald viscometer (the same applies below). At a kinematic viscosity greater than 2 $mm^2/s$, silicone oil does not readily volatize at 25°C and remains in the film, giving rise to a sticky sensation when the oil-in-water emulsion composition is included in cosmetic preparations and the like. Examples of silicone oils include those of the following general formulae.

[Chem. 1]

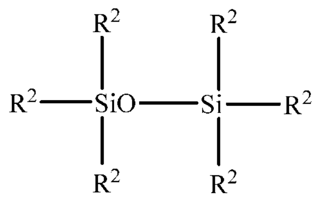

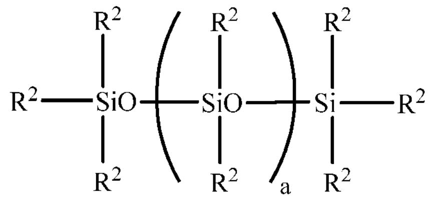

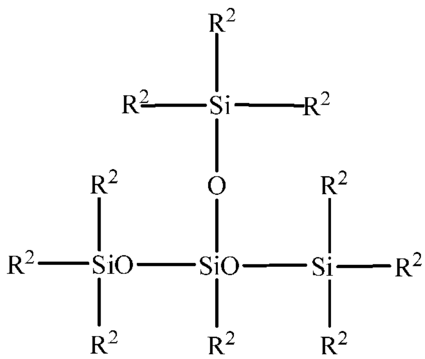

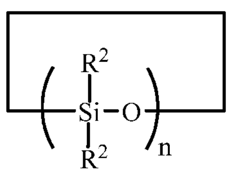

In these formulae, the subscript "a" is an integer from 1 to 3, each $R^2$ is a like or unlike monovalent hydrocarbon group or 1 to 10 carbon atoms, and n is an integer from 4 to 6.

$R^2$ is exemplified in the same way as R above.

Hydrocarbon oils are exemplified by volatile hydrocarbon oils that are linear or branched. Specific examples include isododecane, α-olefin oligomers, light isoparaffin, light liquid isoparaffin, liquid paraffin and liquid isoparaffin. These should be suitably selected according to, for example, the desired feeling upon use. However, from the standpoint of versatility, isododecane and isoparaffin are preferred.

Examples of ester oils include ethyl acetate, butyl acetate and isopropyl acetate.

The weight ratio between the silicone resin (A-1) and the volatile oil (A-2), expressed as (A-1)/(A-2), is from 70/30 to 92/8, and preferably from 80/20 to 90/10. At a volatile oil (A-2) proportion higher than 70/30, when a cosmetic preparation containing the oil-in-water emulsion composition is applied, cracking arises; also, the volatile oil (A-2) does not volatilize quickly and may interfere with the properties of the cosmetic preparation or irritate the skin. On the other hand, at a silicone resin (A-1) proportion higher than 92/8, the storage stability decreases and concentration separation may arise over time.

The silicone resin composition (A) has a kinematic viscosity at 25°C of from 100 to 4,000 $mm^2/s$, and preferably from 200 to 2,000 $mm^2/s$. At a silicone resin composition (A) kinematic viscosity below 100 $mm^2/s$, emulsification with an ordinary agitator such as a homogenizing mixer or a homogenizing disperser may be difficult, emulsion particles of a small size may not be obtained in the oil-in-water emulsion composition, the shelf stability may decrease, and concentration separation may arise over time. At a silicone resin composition (A) kinematic viscosity greater than 4,000 $mm^2/s$, the shelf stability of the inventive oil-in-water emulsion composition may decrease and concentration separation may arise over time.

COMPONENT (B)

Component (B) is a polyoxyethylene sorbitan fatty acid ester having an average number of added moles of ethylene oxide (EO) of at least 10 and a hydrophilic-lipophilic balance (HLB) of at least 11. It may be used singly or two or more may be used in suitable combination. By using such a sorbitan fatty acid ester, an oil-in-water emulsion composition that is mild to the skin and has good coating properties and shelf stability can be achieved.

Illustrative examples of the polyoxyethylene sorbitan fatty acid ester include polyoxyethylene sorbitan monolaurate, polyoxyethylene sorbitan monostearate, polyoxyethylene sorbitan monooleate, polyoxyethylene sorbitan monopalmitate, polyoxyethylene sorbitan isostearate, polyoxyethylene sorbitan triooleate and polyoxyethylene sorbitan tristearate. Of these, in terms of emulsifiability, polyoxyethylene sorbitan monolaurate, polyoxyethylene sorbitan monostearate, polyoxyethylene sorbitan monooleate and polyoxyethylene sorbitan monopalmitate are preferred. Polyoxyethylene sorbitan monostearate and polyoxyethylene sorbitan monooleate are more preferred because they are recognized as food additives.

The number of moles of ethylene oxide added is at least 10, preferably from 10 to 100, and more preferably from 15 to 80. When the number of moles of ethylene oxide added is less than 10, the surfactant tends to remain in the applied film of silicone resin (A-1); hence, there may be a decline in water repellency and stickiness may arise.

The HLB is at least 11, preferably from 13 to 18, and more preferably from 14 to 17. The HLB is determined by Griffin's formula. When a liquid surfactant having an HLB of less than 11 is included, the surfactant tends to remain in the applied film of silicone resin (A-1); hence, there may be a decline in water repellency and stickiness may arise.

In general, surfactants which have a low molecular weight or which have functional groups such as amino groups or include much salt are known to give rise to irritation of the skin. Therefore, to further reduce irritation by the oil-in-water emulsion composition of the invention, it is desirable to select a polyoxyethylene sorbitan fatty acid ester having an even lower content of salt and low-molecular-weight compounds.

COMPONENT (C)

Purified water or the like may be suitably selected and used as the water.

COMPONENT (D)

For the reasons given below, it is preferable for the oil-in-water emulsion composition of the invention to include (D) one or more ingredients selected from the group consisting of 1,3-butylene glycol, glycerin, maltitol, sorbitol, 1,2-pentanediol, 1,2-hexanediol, polyethylene glycol and trimethylglycine. Component (D) may be optionally included in the oil-in-water emulsion composition. One type may be used alone or two or more types may be used in suitable combination. Of these, from the standpoint of versatility, 1,3-butylene glycol, glycerin and polyethylene glycol are preferred.

By thus including component (D), when component (A) is emulsified, emulsion particles of the oil-in-water emulsion composition become finer and the shelf stability further improves. Moreover, because component (D) is a humectant, including component (D) enhances the moisture-retaining ability of the oil-in-water emulsion composition and, when the emulsion composition is included in a cosmetic preparation and applied to the skin, is able to impart a moisturizing effect.

COMPONENT (E)

From the standpoint of holding down film stickiness, increasing film strength, enhancing longevity when included

in cosmetic preparations, increasing emulsifiability and thickening the oil-in-water emulsion composition and thus increasing the shelf stability, it is preferable to include (E) a water-soluble polymeric compound in the oil-in-water emulsion composition of the invention. "Water-soluble polymeric compound" refers to a polymeric compound which has polar groups on the molecule and is dispersible in water. The water-soluble polymeric compound may be a naturally occurring polymeric compound, a semi-synthetic product or a synthetic product. Specific examples include starch, mannan, galactan from seaweeds, alginates, gum arabic, dextran, gelatin, casein, collagen, polyvinyl alcohol, methylcellulose, carboxymethylcellulose hydroxymethylcellulose, polyvinyl pyrrolidone, xanthan gum and acrylic acid polymers. These may be used singly or two or more may be used in suitable combination.

COMPONENT (F)

In terms of film stickiness and increasing the longevity of cosmetic preparations, it is preferable to include (F) a synthetic non-crystalline silica in the oil-in-water emulsion composition of the invention. The synthetic non-crystalline silica may be a dry silica obtained by a combustion method that burns silicon tetrachloride in oxygen and hydrogen, or a wet silica obtained by a precipitation method or gel method that neutralizes sodium silicate with sulfuric acid or a sol-gel method that hydrolyses an alkoxysilane. The synthetic non-crystalline silica may be of one type used alone or two or more types may be used in suitable combination. The synthetic non-crystalline silica has a primary particle size that is preferably from 1 to 50 nm, and a BET specific surface area that is preferably from 50 to 1,000 $m^2/g$. Measurements for the primary particles of the synthetic non-crystalline silica are equivalent values obtained from specific surface area measurements by the BET adsorption method (in accordance with JIS Z8830). When the synthetic non-crystalline silica is included directly as is in the oil-in-water emulsion composition of the invention, by either including it when emulsifying the silicone resin composition (A) or first including it in the silicone resin composition (A) and subsequently emulsifying, the synthetic non-crystalline silica can be uniformly dispersed, making it difficult for concentration separation to arise within the oil-in-water emulsion composition. When the synthetic non-crystalline silica is included after dispersion in water, that is, as colloidal silica, addition may be carried out either when the silicone resin composition (A) is emulsified or following emulsification. From the standpoint of the ease of addition as a synthetic non-crystalline silica, colloidal silica is preferred.

Specific examples of component (F) include the following products. Powdery dry silicas include HDK V15, HDK N20, HDK T30, HDK T40, HDK H15, HDK H18, HDK H20 and HDK H30 from Wacker Asahi Kasei Silicone Co., Ltd.; and AEROSIL 50, AEROSIL 90G, AEROSIL 130, AEROSIL 200, AEROSIL 200CF, AEROSIL 200V, AEROSIL 200FAD, AEROSIL 300, AEROSIL 300CF, AEROSIL 380, AEROSIL OX50 and AEROSIL TT600 from Nippon Aerosil Co., Ltd.. Powdery wet silicas include NIPSIL, NIPGEL AZ-200, NIPGEL AZ-201, NIPGEL AZ-410, NIPGEL AZ-260, NIPGEL AZ-360, NIPGEL AZ-460, NIPGEL AZ-6A0, NIPGEL AY-200, NIPGEL AY-220, NIPGEL AY-420, NIPGEL AY-601, NIPGEL AY-6A3, NIPGEL AY-8A2, NIPGEL BY-001, NIPGEL BY-200, NIPGEL BY-400, NIPGEL BY-601, NIPGEL CX-200, NIPGEL CX-600, NIPGEL CY-200, NIPSIL VN3, NIPSIL AQ, NIPSIL LP, NIPSIL NA, NIPSIL ER, NIPSIL ER-R, NIPSIL RS-150, NIPSIL AQ, NIPSIL NS, NIPSIL NS-T, NIPSIL NS-K, NIPSIL NS-KR, NIPSIL NA, NIPSIL KP, NIPSIL L-300, NIPSIL KQ, NIPSIL NS-P, NIPSIL E-200A, NIPSIL E-220A, NIPSIL K=500, NIPSIL E-1009, NIPSIL E-1011, NIPSIL E-1030, NIPSIL E-150J, NIPSIL E-170, NIPSIL E-200, NIPSIL E-220, NIPSIL E-743, NIPSIL E-75, NIPSIL HD, NIPSIL HD-2, NIPSIL G-300, NIPSIL E-74P and NIPSIL N-300A from Tohso Silica Corporation. Examples of colloidal silicas that are aqueous dispersions of synthetic non-crystalline silicas include BINZIL 15/500, BINZIL 30/360, BINZIL 30/220, NIZIL 30/80, BINZIL 305, BINZIL 40/220, BINZIL 40/130, BINZIL 50/80, NYACOL 215, NYACOL 830, NYACOL 1430, NYACOL 1440, NYACOL 2040, NYACOL 2050 and NYACOL 9950 From AkzoNobel N.V; SNOWTEX 20, SNOWTEX 30, SNOWTEX 40, SNOWTEX C, SNOWTEX N, SNOWTEX O, SNOWTEX S, SNOWTEX 20L and SNOWTEX OL from Nissan Chemical Industries, Ltd.; and COSMO S-40 from JGC Catalysts and Chemicals Ltd.

The contents of the various ingredients in the oil-in-water emulsion composition are described below.

The component (A) content is from 30 to 70 wt%, and preferably from 40 to 60 wt%. At a content lower than 30 wt%, properties such as longevity and water repellency when the emulsion composition is included in cosmetic preparations are difficult to obtain. On the other hand, at a content greater than 70 wt%, the viscosity of the oil-in-water emulsion composition of the invention rises and handling becomes difficult.

The component (B) content is from 2 to 10 wt%, and preferably from 3 to 7 wt%. At a content of polyoxyethylene sorbitan fatty acid ester (B) below 2 wt%, emulsion particles of the oil-in-water emulsion composition may not achieve a small particle size, shelf stability may decline and concentration separation may arise. Also, when the oil-in-water emulsion composition is applied, crawling may arise. On the other hand, at a content above 10 wt%, the polyoxyethylene sorbitan fatty acid ester (B) may interfere with the properties of the silicone resin (A-1), which may lower the longevity and water repellency of the cosmetic preparation.

The component (C) content is from 20 to 62 wt%, and preferably from 30 to 55 wt%. At less than 20 wt%, the viscosity of the oil-in-water emulsion composition of the invention may increase, making handleability more difficult. On the other hand, at more than 62 wt%, water (C) tends to remain, which may lower the longevity and water-repellency of the cosmetic preparation.

Component (D) is an optional ingredient. When included, the content is preferably from 0.1 to 5%, and more preferably from 0.2 to 3 wt%. At a component (D) content of 0.1 wt% or more, the advantageous effects of including component (D) can be obtained. On the other hand, a content of more than 5 wt% may interfere with the properties of the silicone resin (A-1), which may lower the longevity and water-repellency of the cosmetic preparation.
Component (E) is an optional ingredient. When included, the content is preferably from 0.1 to 5%, and more preferably from 0.2 to 3 wt%. At a component (E) content of 0.1 wt% or more, the advantageous effects of including component (E) can be obtained. On the other hand, a content of more than 5 wt% may interfere with the water-repellency of the cosmetic preparation.
Component (F) is an optional ingredient. When included, the content is preferably from 0.1 to 10%, and more preferably from 0.2 to 5 wt%. At a component (F) content of 0.1 wt% or more, the advantageous effects of including component (F) can be obtained. However, at content of more than 10 wt%, cracks may arise in the film. Various other ingredients may be included in the oil-in-water emulsion composition of the invention within ranges that do not detract from the advantageous effects of the invention when used in ordinary cosmetic preparations.

## PRODUCTION METHOD

The production method of this invention includes agitating and thereby emulsifying a composition containing the above (A) silicone resin composition, (B) polyoxyethylene sorbitan fatty acid ester and (C) water (Step (I)). More preferred examples are described in detail below.

(I) Emulsification Step

Components (A), (B) and (C) are added together and emulsification is carried out by high-speed stirring with a homogenizing mixer or a homogenizing disperser. When the entire amounts of components (A), (B) and (C) are added all at once and emulsification is carried out, fine emulsion particles may not be obtained or emulsification may not take place. In cases where emulsification does not take place or fine emulsion particles are not obtained, emulsification can be easily carried out and an oil-in-water emulsion composition having small emulsion particles and a good shelf stability obtained by adding some of components (A), (B) and (C) and emulsifying, subsequently adding the remainder of components (A) and (B) and emulsifying, then high-speed stirring with a homogenizing mixer or a homogenizing disperser until the target emulsion particle size is achieved, and finally adding the remainder of component (C) to effect dilution.
The emulsification temperature is not particularly limited, although it is desirable to set it at or below the flash point of the oil-in-water emulsion composition of the invention. The emulsification temperature is preferably between 0°C and 80°C, and more preferably between 0°C and 40°C. By setting the emulsification temperature to between 0°C and 80°C, emulsification is easy and the emulsion composition becomes more stable. The stirring speed is preferably from 100 to 10,000 rpm, and more preferably from 500 to 5,000 rpm. The emulsification time is not particularly limited, although it is preferably from 1 to 240 minutes when production is carried out with a batch-type emulsifier, and is preferably 1 minute or less when production is carried out with a continuous emulsifier. The pressure during emulsification is not limited to normal pressure; that is, emulsification may also be carried out under reduced pressure or applied pressure. When agitation is carried out under reduced pressure or applied pressure, this discourages the admixture of bubbles, enabling more effective emulsification. When the system is set to a reduced pressure, this pressure is made higher than the vapor pressure of the starting materials so as to prevent volatilization of the starting materials.
Emulsifiers that may be used include, for example, the Homogenizing Mixer (Primix Corporation), the Homogenizing Disper (Primix Corporation), the Agi Homo Mixer (Primix Corporation), the Combi Mix (Primix Corporation)--which is a three-shaft dispersion mixer that combines the Homogenizing Mixer, the Homogenizing Disper and the Anchor Mixer, colloid mills having an agitating mechanism consisting of a rotor and a stator (such as those available from IKA, PUC, Nissei Corporation and Iwaki Co., Ltd.), high-shear mixers (such as those available from Silverson and Primix Corporation) and the Hivis Disper Mix model 3D-5 (Primix Corporation), which is an agitator based on the orbital revolution and own-axis rotation of two blades and the high-speed rotation of a toothed blade.
The absolute viscosity of the oil-in-water emulsion composition, although not particularly specified, is preferably from 5 to 20,000 mPa·s, more preferably from 10 to 5,000 mPa·s, and even more preferably from 20 to 2,000 mPa·s. At an absolute viscosity of less than 5 mPa·s, the shelf stability may decrease and cracking may arise. On the other hand, at an absolute viscosity greater than 20,000 mPa·s, handling may be difficult. The absolute viscosity of the oil-in-water emulsion composition is a value measured at 25°C with a BM-type rotational viscometer.
The emulsion particles in the oil-in-water emulsion composition have an average size of preferably 400 nm or less, and more preferably 350 nm or less. When the average particle size is larger than 400 nm, separation may soon arise. Although the average particle size has no particular lower limit, it may be set to generally at least 100 nm, and especially at least 150 mm. The average particle size is measured by a dynamic light scattering method or a laser diffraction method. In this invention, the average particle size is the volume mean particle size (cumulative mean diameter $D_{50}$ (median

diameter)) obtained by the laser diffraction method. The instrument used for the dynamic light scattering method is exemplified by the N4 PLUS (BECKMAN COULTER), the DelsaMax CORE (BECKMAN COULTER) and the DelsaMax Pro (BECKMAN COULTER). The instrument used for the laser diffraction method is exemplified by the LA920 and the LA960 (both from Horiba, Ltd.).

COSMETIC PREPARATIONS

The oil-in-water emulsion compositions of the invention may be included in skin care cosmetics, hair care cosmetics and the like. Based on the properties of the invention, inclusion in makeup cosmetics and skin care cosmetics is especially preferred. Examples of makeup cosmetics include foundation (including all solid and liquid foundations), creams, makeup bases, skin milks, eyeshadows, lipsticks, lip creams, rouges, eyebrows, mascaras, eyeliners, cleansing preparations and packs. The form of the cosmetic preparation is not particularly limited. For example, various forms such as liquids, emulsions, creams, solids, pastes, gels, multilayer preparations, mousses, sprays, sticks and pencils may be selected. The content of the oil-in-water emulsion composition therein is preferably from 1 to 70 wt%, and more preferably from 3 to 50 wt%, of the cosmetic preparation. The advantageous effects of the invention are readily obtained particularly within this range. When the content is too high, a heavy texture may result.

Various ingredients used in conventional cosmetic preparations may be included in the cosmetic preparations of the invention, within ranges that do not detract from the advantageous effects of the invention. For example, the following may be included as ingredients: oils, alcoholic hydroxyl group-containing compounds, surfactants, powders, compositions of a crosslinked organopolysiloxane and an oil that is liquid at room temperature, silicone waxes, film-forming agents, antiperspirants, antimicrobial agents and other additives. These may be used singly or two or more may be used in suitable combination.

EXAMPLES

The invention is illustrated more fully below by way of Examples and Comparative Examples. In the following Examples, unless noted otherwise, references to "%" in a composition signify percent by weight, and references to "ratio" signify a weight ratio. Kinematic viscosities are measured values obtained at 25°C with an Ostwald viscometer.

Component (A) and component (B) used in the Examples and the Comparative Examples are described below.

(A-1) Silicone Resins

· (A-1 [1])

The silicone resin represented as $[(CH_3)_3SiO_{0.5}]_{0.54}[SiO_2]_{0.46}$ (molecular weight, 2,000; absolute viscosity at 36°C, 20,000 mPa·s; liquid; SiOH group content, 0.08 mol/g)

$$[R_3SiO_{0.5}]\text{ units}/[SiO_2]\text{ units} = 1.2$$

· (A-1 [2])

The silicone resin represented as $[(CH_3)_3SiO_{0.5}]_{0.57}[SiO_2]_{0.43}$ (molecular weight, 1,800; absolute viscosity at 36°C, 10,000 mPa·s; liquid; SiOH group content, 0.03 mol/g)

$$[R_3SiO_{0.5}]\text{ units}/[SiO_2]\text{ units} = 1.3$$

· (A-1 [3])

The silicone resin represented as $[(CH_3)_3SiO_{0.5}]_{0.41}[SiO_2]_{0.59}$ (molecular weight, 15,000; solid at 36°C; SiOH group content, 0.05 mol/g)

$$[R_3SiO_{0.5}]\text{ units}/[SiO_2]\text{ units} = 0.7$$

The weight-average molecular weight is the polystyrene-equivalent weight-average molecular weight obtained by gel permeation chromatography. The absolute viscosity is a value measured with a BM-type rotational viscometer.

(A-2) Volatile Oils

- (A-2 [1]): Decamethylcyclopentasiloxane (kinematic viscosity, 4 $mm^2/s$)
- (A-2 [2]): Isododecane (kinematic viscosity, 1.4 $mm^2/s$)

(A) Silicone Resin Compositions

- (A [1])

$$(A\text{-}1\ [1])/(A\text{-}2\ [1]) = 90/10;$$

kinematic viscosity, 1,100 $mm^2/s$

- (A [2])

$$(A\text{-}1\ [1])/(A\text{-}2\ [2]) = 90/10;$$

kinematic viscosity, 1,200 $mm^2/s$

- (A [3])

$$(A\text{-}1\ [2])/(A\text{-}2\ [2]) = 90/10;$$

kinematic viscosity, 400 $mm^2/s$

- (A [4])

$$(A\text{-}1\ [1])/(A\text{-}2\ [2]) = 80/20;$$

kinematic viscosity, 100 $mm^2/s$

- (A [5]) Comparative Product

$$(A\text{-}1\ [1])/(A\text{-}2\ [2]) = 60/40;$$

kinematic viscosity, 20 $mm^2/s$

- (A [6]) Comparative Product

$$(A\text{-}1\ [3])/(A\text{-}2\ [1]) = 50/50;$$

kinematic viscosity, 1,900 $mm^2/s$

- (A [7]) Comparative Product

$$(A\text{-}1\ [1])/(A\text{-}2\ [2]) = 95/5;$$

kinematic viscosity, 4,500 $mm^2/s$

(B) Polyoxyethylene Sorbitan Fatty Acid Esters

· (B [1])

Nonion OT-80
(polyoxyethylene sorbitan monooleate (20 E.O.); HLB, 15.7)

· (B [2])

Nonion ST-60
(polyoxyethylene sorbitan monooleate (20 E.O.); HLB, 15.7; NOF Corporation)

· Surfactant [3] Comparative Product

NIKKOL Decaglyn 1-LV EX
(polyglyceryl-10 laurate; HLB, 15.5; Nikko Chemical Co., Ltd.)

· Surfactant [4] Comparative Product

NIKKOL Decaglyn 1-SV EX
(polyglyceryl-10 stearate; HLB, 12.5; Nikko Chemical Co., Ltd.)

(B [1]), (B [2]), (B [3]) and (B [4]) are ingredients having the same structures as ingredients that have been recognized by the Japanese Ministry of Health, Labour and Welfare as food additives.

Example 1

Seventy-five grams of Silicone Resin Composition (A [1]), 3 g of Polyoxyethylene Sorbitan Fatty Acid Ester (B [1]) and 17 g of water (C) were added together, then emulsified by stirring for 3 minutes at 3,000 rpm with the T.K. Homogenizing Mixer (Primix Corporation). Another 75 g of Silicone Resin Composition (A [1]) was added and 3 minutes of stirring was carried out at 2,000 rpm with the T.K. Homogenizing Mixer (Primix Corporation), following which 16.5 g of Polyoxyethylene Sorbitan Fatty Acid Ester (B [1]) was added and stirring was carried out with the T.K. Homogenizing Disper (Primix Corporation), first at 500 rpm for 3 minutes and then at 1,500 rpm for 20 minutes. Next, 113.5 g of water (C) was added and 3 minutes of stirring was carried out at 1,500 rpm with the T.K. Homogenizing Mixer (Primix Corporation), giving Oil-in-Water Emulsion Composition (1). Emulsion particles in Emulsion Composition (1) had an average particle size of 270 nm, and the absolute viscosity was 110 mPa·s.

Example 2

Seventy-five grams of Silicone Resin Composition (A [2]), 3 g of Polyoxyethylene Sorbitan Fatty Acid Ester (B [1]) and 17 g of water (C) were added together, then emulsified by stirring for 3 minutes at 3,000 rpm with the T.K. Homogenizing Mixer (Primix Corporation). Another 75 g of Silicone Resin Composition (A [2]) was added and 3 minutes of stirring was carried out at 2,000 rpm with the T.K. Homogenizing Mixer (Primix Corporation), following which 16.5 g of Polyoxyethylene Sorbitan Fatty Acid Ester (B [1]) was added and stirring was carried out with the T.K. Homogenizing Disper (Primix Corporation), first at 500 rpm for 3 minutes and then at 1,500 rpm for 20 minutes. Next, 113.5 g of water (C) was added and 3 minutes of stirring was carried out at 1,500 rpm with the T.K. Homogenizing Mixer (Primix Corporation), giving Oil-in-Water Emulsion Composition (2). Emulsion particles in Emulsion Composition (2) had an average particle size of 260 nm, and the absolute viscosity was 120 mPa·s.

Example 3

Seventy-five grams of Silicone Resin Composition (A [2]), 3 g of Polyoxyethylene Sorbitan Fatty Acid Ester (B [2]) and 17 g of water (C) were added together, then emulsified by stirring for 3 minutes at 3,000 rpm with the T.K. Homogenizing Mixer (Primix Corporation). Another 75 g of Silicone Resin Composition (A [2]) was added and 3 minutes of stirring was carried out at 2,000 rpm with the T.K. Homogenizing Mixer (Primix Corporation), following which 16.5 g of Polyoxyethylene Sorbitan Fatty Acid Ester (B [2]) was added and stirring was carried out with the T.K. Homogenizing

Disper (Primix Corporation), first at 500 rpm for 3 minutes and then at 1,500 rpm for 20 minutes. Next, 113.5 g of water (C) was added and 3 minutes of stirring was carried out at 1,500 rpm with the T.K. Homogenizing Mixer (Primix Corporation), giving Oil-in-Water Emulsion Composition (3). Emulsion particles in Emulsion Composition (3) had an average particle size of 260 nm, and the absolute viscosity was 150 mPa·s.

Example 4

Seventy-five grams of Silicone Resin Composition (A [3]), 3 g of Polyoxyethylene Sorbitan Fatty Acid Ester (B [1]) and 17 g of water (C) were added together, then emulsified by stirring for 3 minutes at 3,000 rpm with the T.K. Homogenizing Mixer (Primix Corporation). Another 75 g of Silicone Resin Composition (A [3]) was added and 3 minutes of stirring was carried out at 2,000 rpm with the T.K. Homogenizing Mixer (Primix Corporation), following which 16.5 g of Polyoxyethylene Sorbitan Fatty Acid Ester (B [1]) was added and stirring was carried out with the T.K. Homogenizing Disper (Primix Corporation), first at 500 rpm for 3 minutes and then at 1,500 rpm for 20 minutes. Next, 113.5 g of water (C) was added and 3 minutes of stirring was carried out at 1,500 rpm with the T.K. Homogenizing Mixer (Primix Corporation), giving Oil-in-Water Emulsion Composition (4). Emulsion particles in Emulsion Composition (4) had an average particle size of 250 nm, and the absolute viscosity was 180 mPa·s.

Example 5

Seventy-five grams of Silicone Resin Composition (A [2]), 3 g of Polyoxyethylene Sorbitan Fatty Acid Ester (B [1]) and 19 g of water (C) were added together, then emulsified by 3 minutes of stirring at 3,000 rpm with the T.K. Homogenizing Mixer (Primix Corporation). Another 75 g of Silicone Resin Composition (A [2]) was added and stirring was carried out at 2,000 rpm for 3 minutes with the T.K. Homogenizing Mixer (Primix Corporation), then 16.5 g of Polyoxyethylene Sorbitan Fatty Acid Ester (B [1]) was added and stirring was carried out with the T.K. Homogenizing Disper (Primix Corporation) at 500 rpm for 3 minutes, following which 3 g of 1,3-butylene glycol (D) was added and stirring was carried out with the T.K. Homogenizing Disper (Primix Corporation) at 500 rpm for 3 minutes. This was followed by 20 minutes of stirring at 1,500 rpm with the T.K. Homogenizing Disper (Primix Corporation). Next, 108.5 g of water (C) was added and 3 minutes of stirring was carried out at 1,500 rpm with the T.K. Homogenizing Mixer (Primix Corporation), giving Oil-in-Water Emulsion Composition (5). Emulsion particles in Emulsion Composition (5) had an average particle size of 250 nm, and the absolute viscosity was 200 mPa·s.

Example 6

Seventy-five grams of Silicone Resin Composition (A [2]), 3 g of Polyoxyethylene Sorbitan Fatty Acid Ester (B [1]) and 17 g of water (C) were added together, then emulsified by stirring for 3 minutes at 3,000 rpm with the T.K. Homogenizing Mixer (Primix Corporation). Another 75 g of Silicone Resin Composition (A [2]) was added and 3 minutes of stirring was carried out at 2,000 rpm with the T.K. Homogenizing Mixer (Primix Corporation), following which 16.5 g of Polyoxyethylene Sorbitan Fatty Acid Ester (B [1]) was added and stirring was carried out with the T.K. Homogenizing Disper (Primix Corporation), first at 500 rpm for 3 minutes and then at 1,500 rpm for 20 minutes. Next, 83.5 g of water (C) was added and 3 minutes of stirring was carried out at 1,500 rpm with the T.K. Homogenizing Mixer (Primix Corporation), following which 30 g of (E) polyvinyl alcohol (10% Gohsenol EG-40C (from Nippon Synthetic Chemical Industry Co., Ltd.)) was added and 3 minutes of stirring was carried out at 1,500 rpm, giving Oil-in-Water Emulsion Composition (6). Emulsion particles in Emulsion Composition (6) had an average particle size of 260 nm, and the absolute viscosity was 1,000 mPa·s.

Example 7

Seventy-five grams of Silicone Resin Composition (A [2]), 3 g of Polyoxyethylene Sorbitan Fatty Acid Ester (B [1]) and 17 g of water (C) were added together, then emulsified by stirring for 3 minutes at 3,000 rpm with the T.K. Homogenizing Mixer (Primix Corporation). Another 75 g of Silicone Resin Composition (A [2]) was added and 3 minutes of stirring was carried out at 2,000 rpm with the T.K. Homogenizing Mixer (Primix Corporation), following which 16.5 g of Polyoxyethylene Sorbitan Fatty Acid Ester (B [1]) was added and stirring was carried out with the T.K. Homogenizing Disper (Primix Corporation), first at 500 rpm for 3 minutes and then at 1,500 rpm for 20 minutes. Next, 98.5 g of water (C) was added and 3 minutes of stirring was carried out at 1,500 rpm with the T.K. Homogenizing Mixer (Primix Corporation). Fifteen grams of (F) colloidal silica alcohol (a 40% aqueous dispersion of colloidal silica (COSMO S-40, from JGC Catalysts and Chemicals Ltd.)) was then added and 3 minutes of stirring was carried out at 1,500 rpm, giving Oil-in-Water Emulsion Composition (7). Emulsion particles in Emulsion Composition (7) had an average particle size of 260 nm, and the absolute viscosity was 80 mPa·s.

Example 8

Seventy-five grams of Silicone Resin Composition (A [4]), 3 g of Polyoxyethylene Sorbitan Fatty Acid Ester (B [2]) and 23 g of water (C) were added together, then emulsified by stirring for 3 minutes at 3,000 rpm with the T.K. Homogenizing Mixer (Primix Corporation). Another 75 g of Silicone Resin Composition (A [4]) was added and 3 minutes of stirring was carried out at 2,000 rpm with the T.K. Homogenizing Mixer (Primix Corporation). Next, 16.5 g of Polyoxyethylene Sorbitan Fatty Acid Ester (B [2]) was added while stirring at 500 rpm with the T.K. Homogenizing Disper (Primix Corporation), following which 50 minutes of stirring was carried out at 1,500 rpm with the T.K. Homogenizing Disper (Primix Corporation). Next, 107.5 g of water (C) was added and 3 minutes of stirring was carried out at 1,500 rpm with the T.K. Homogenizing Mixer (Primix Corporation), giving Oil-in-Water Emulsion Composition (8). Emulsion particles in Emulsion Composition (8) had an average particle size of 260 nm, and the absolute viscosity was 200 mPa·s.

Comparative Example 1

Seventy-five grams of Silicone Resin Composition (A [5]), 3 g of Polyoxyethylene Sorbitan Fatty Acid Ester (B [1]) and 23 g of water (C) were added together, then emulsified by stirring for 3 minutes at 500 rpm with the T.K. Homogenizing Mixer (Primix Corporation). Seventy-five grams of Silicone Resin Composition (A [4]) was added and 3 minutes of stirring was carried out at 2,000 rpm with the T.K. Homogenizing Mixer (Primix Corporation). Next, 16.5 g of Polyoxyethylene Sorbitan Fatty Acid Ester (B [1]) was added and stirring was carried out with the T.K. Homogenizing Disper (Primix Corporation), first at 500 rpm for 3 minutes then at 1,500 rpm for 20 minutes. Next, 107.5 g of water (C) was added and 3 minutes of stirring was carried out at 1,500 rpm with the T.K. Homogenizing Mixer (Primix Corporation), giving Oil-in-Water Emulsion Composition (9). Emulsion particles in Emulsion Composition (9) had an average particle size of 400 nm, and the absolute viscosity was 300 mPa·s.

Comparative Example 2

Seventy-five grams of Silicone Resin Composition (A [6]), 3 g of Polyoxyethylene Sorbitan Fatty Acid Ester (B [1]) and 17 g of deionized water (C) were added together, then emulsified by stirring for 3 minutes at 1,000 rpm with the T.K. Homogenizing Mixer (Primix Corporation). Seventy-five grams of Silicone Resin Composition (A [5]) was added and 3 minutes of stirring was carried out at 2,000 rpm with the T.K. Homogenizing Mixer (Primix Corporation). Next, 16.5 g of Polyoxyethylene Sorbitan Fatty Acid Ester (B [1]) was added and stirring was carried out with the T.K. Homogenizing Disper (Primix Corporation), first at 500 rpm for 3 minutes then at 1,500 rpm for 20 minutes. Next, 113.5 g of water (C) was added and 3 minutes of stirring was carried out at 1,500 rpm with the T.K. Homogenizing Mixer (Primix Corporation), giving Oil-in-Water Emulsion Composition (10). Emulsion particles in Emulsion Composition (10) had an average particle size of 300 nm, and the absolute viscosity was 80 mPa·s.

Comparative Example 3

Seventy-five grams of Silicone Resin Composition (A [7]), 3 g of Polyoxyethylene Sorbitan Fatty Acid Ester (B [1]) and 17 g of water (C) were added together, then emulsified by stirring for 3 minutes at 3,000 rpm with the T.K. Homogenizing Mixer (Primix Corporation). Seventy-five grams of Silicone Resin Composition (A [6]) was added and 3 minutes of stirring was carried out at 2,000 rpm with the T.K. Homogenizing Mixer (Primix Corporation). Next, 16.5 g of Polyoxyethylene Sorbitan Fatty Acid Ester (B [1]) was added and stirring was carried out with the T.K. Homogenizing Disper (Primix Corporation), first at 500 rpm for 3 minutes then at 1,500 rpm for 20 minutes. Next, 113.5 g of water (C) was added and 5 minutes of stirring was carried out at 1,500 rpm with the T.K. Homogenizing Mixer (Primix Corporation), giving Oil-in-Water Emulsion Composition (11). Emulsion particles in Emulsion Composition (11) had an average particle size of 270 nm, and the absolute viscosity was 150 mPa·s.

Comparative Example 4

Seventy-five grams of Silicone Resin Composition (A [2]), 1.8 g of Surfactant [3], 1.2 g of Surfactant [4] and 17 g of water (C) were added together, then emulsified by stirring for 3 minutes at 3,000 rpm with the T.K. Homogenizing Mixer (Primix Corporation). Another 75 g of Silicone Resin Composition (A [2]) was added and 3 minutes of stirring was carried out at 2,000 rpm with the T.K. Homogenizing Mixer (Primix Corporation). Next, 9.9 g of surfactant [3] and 6.6 g of surfactant [4] were added and stirring was carried out with the T.K. Homogenizing Disper (Primix Corporation), first at 500 rpm for 3 minutes then at 1,500 rpm for 20 minutes. Next, 113.5 g of water (C) was added and 3 minutes of stirring was carried out at 1,500 rpm with the T.K. Homogenizing Mixer (Primix Corporation), giving Oil-in-Water Emulsion Composition (12). Emulsion particles in Emulsion Composition (12) had an average particle size of 350 nm, and the absolute viscosity was 50

mPa·s.

The ingredient makeup (%), average particle size, viscosity and results of evaluations on stability to centrifugation, coatability, and tendency to crack of Oil-in-Water Emulsion Compositions 1 to 12 obtained in the above Examples are shown in the Tables below. The average particle sizes are measured values obtained with the HORIBA LA920 (Horiba, Ltd.), and the composition absolute viscosities are values measured at 25°C with a BM-type rotational viscometer.

Stability to Centrifugation

About 25 g of the emulsion composition (Emulsion Compositions 1 to 12) was placed in a centrifuge tube and 50 minutes of centrifugation was carried out at 3,000 rpm in the H-19FM centrifuge (Kokusan Co., Ltd.), following which the nonvolatile contents (105°C, 3 hours) of the bottom and top layers were measured. The results are shown as the value obtained by dividing the nonvolatiles content of the top layer by the nonvolatiles content of the bottom layer. Values obtained by dividing the nonvolatiles content of the top layer by the nonvolatiles content of the bottom layer are acceptable when they fall in the range of 0.95 to 1.05. The more such a value differs from unity (1), the greater the likelihood of separation occurring over time.

Coating Ability

Emulsion Compositions 1 to 12 were applied (film thickness, approx. 20 μm) onto uncoated paperboard (15 cm × 7 cm) using a No. 13 bar coater, following which they were dried at 36°C for 36 hours and evaluated for crawling.

Rating Criteria for Coating Ability

◦: No crawling, or crawling occurs on less than 30% of overall coated surface
△: Crawling occurs on at least 30% but less than 50% of overall coated surface
×: Crawling occurs on at least 50% of overall coated surface
A rating of "△" or "◦" is acceptable.

Tendency to Crack

The paperboard used to evaluate coatability was wound onto a tube having a diameter of 3.5 cm, and cracking was evaluated.

Rating Criteria for Tendency to Crack

◦: No cracks
×: Some cracks

Table 1

| Composition (%) | | | Example | | | | | | | |
|---|---|---|---|---|---|---|---|---|---|---|
| | | | 1 | 2 | 3 | 4 | 5 | 6 | 7 | 8 |
| Component (A) | Component (A-1) | [1] | 45 | 45 | 45 | | 45 | 45 | 45 | 40 |
| | | [2] | | | | 45 | | | | |
| | Component (A-2) | [1] | 5 | | | | | | | |
| | | [2] | | 5 | 5 | 5 | 5 | 5 | 5 | 10 |
| Type of Component (A) | | | A [1] | A [2] | A [2] | A [3] | A [2] | A [2] | A [2] | A [4] |
| Component (B) | | [1] | 6.5 | 6.5 | | 6.5 | 6.5 | 6.5 | 6.5 | |
| | | [2] | | | 6.5 | | | | | 6.5 |

(continued)

| Composition (%) | Example | | | | | | | |
|---|---|---|---|---|---|---|---|---|
| | 1 | 2 | 3 | 4 | 5 | 6 | 7 | 8 |
| Component (C) | 43.5 | 43.5 | 43.5 | 43.5 | 42.5 | 42.5 | 41.5 | 43.5 |
| Component (D) | | | | | 1 | | | |
| Component (E) | | | | | | 1 | | |
| Component (F) | | | | | | | 2 | |
| Total | 100 | 100 | 100 | 100 | 100 | 100 | 100 | 100 |
| Emulsion composition | 1 | 2 | 3 | 4 | 5 | 6 | 7 | 8 |
| Average particle size (nm) | 270 | 260 | 260 | 250 | 250 | 260 | 260 | 260 |
| Absolute viscosity (mPa·s) | 110 | 120 | 150 | 180 | 200 | 1,000 | 80 | 250 |
| Stability to centrifugation | 0.97 | 0.97 | 0.97 | 1.00 | 0.98 | 0.98 | 0.95 | 1.05 |
| Coating ability | ○ | ○ | ○ | ○ | ○ | ○ | ○ | O |
| Tendency to crack | ○ | ○ | ○ | ○ | ○ | ○ | ○ | ○ |

Table 2

| Composition (%) | | | Comparative Example | | | |
|---|---|---|---|---|---|---|
| | | | 1 | 2 | 3 | 4 |
| Component (A) | Component (A-1) | [1] | 30 | | 47.5 | 45 |
| | | [3] | | 25 | | |
| | Component (A-2) | [1] | | | | |
| | | [2] | 20 | 25 | 2.5 | 5 |
| Type of Component (A) | | | A [5] | A [6] | A [7] | A [2] |
| Component (B) and Surfactant | | [1] | 6.5 | 6.5 | 6.5 | |
| | | [3] | | | | 3.9 |
| | | [4] | | | | 2.6 |
| Component (C) | | | 43.5 | 43.5 | 43.5 | 43.5 |
| Total | | | 100 | 100 | 100 | 100 |
| Emulsion composition | | | 9 | 10 | 11 | 12 |
| Average particle size (nm) | | | 400 | 300 | 270 | 350 |
| Absolute viscosity (mPa·s) | | | 300 | 80 | 150 | 50 |
| Stability to centrifugation | | | 1.2 | 1.2 | 0.91 | 1.5 |
| Coating ability | | | ○ | ○ | ○ | × |
| Tendency to crack | | | ○ | × | ○ | ○ |

Example 9 Mascara

| | |
|---|---|
| (1) Water | 35 % |
| (2) Bentonite | 4 % |
| (3) 1,3-Butylene glycol | 6 % |
| (4) Preservative | suitable amount |

(continued)

| | |
|---|---|
| (5) Cellulose fibers | 5 % |
| (6) Polyoxyethylene (20) sorbitan monostearate | 4 % |
| (7) Emulsion Composition (2) or Emulsion Composition (8) | 35 % |
| (8) Black iron oxide | 10 % |
| (9) Polvvinvl alcohol | 1 % |
| Total | 100 % |

Method of Preparation

Bentonite (2) was added to part of the water (1) and thoroughly swelled in a homogenizing mixer, following which ingredients (3) to (9) were added and stirring was carried out.

The mascaras in which Emulsion Composition (2) or Emulsion Composition (9) was used had a uniform applied film and long-lasting properties, although the mascara formulated with Emulsion Composition (2) was especially long-lasting.

Example 10 Oil-in-Water Cream

| | |
|---|---|
| (1) Emulsion Composition (2) or Emulsion Composition (9) | 15 % |
| (2) Glyceryl triethylhexanoate | 5 % |
| (3) Dipropylene glycol | 7% |
| (4) Glycerin | 5 % |
| (5) Methylcellulose (2% aqueous solution) 1) | 7% |
| (6) Polyacrylamide emulsifying agent 2) | 2% |
| (7) Preservative | suitable amount |
| (8) Flavor | suitable amount |
| (9) Purified water | suitable amount |
| Total | 100 % |

1) Metolose SM-4000, from Shin-Etsu Chemical Co., Ltd.

2) Sepigel 305, from SEPPIC

Method of Preparation

A: Ingredients (3) to (9) were mixed together.

B: Ingredients (1) and (2) were mixed together, Mixture A was added, and stirring was carried out to effect emulsification.

These creams in which Emulsion Composition (2) or Emulsion Composition (9) was used were not sticky, had a good spreadability when applied and felt as if they adhered well to the skin, although the sense of adherence to the skin was particularly good with the use of Emulsion Composition (2).

## Claims

**1.** An oil-in-water emulsion composition comprising:

(A) 30 to 70 wt% of a silicone resin composition which is a mixture consisting of

(A-1) silicone resin having [$R_3SiO_{0.5}$] units and [$SiO_2$] units in a molar ratio, expressed as [$R_3SiO_{0.5}$]/[$SiO_2$], of from 0.7 to 1.4, wherein each R is independently a monovalent organic group of 1 to 10 carbon atoms, and
(A-2) volatile oil having a boiling point at normal pressure of 260°C or less, at a weight ratio, expressed as (A-1) /(A-2), of from 70/30 to 92/8, and has a kinematic viscosity at 25°C of from 100 to 4,000 $mm^2/s$, as measured with Ostwald viscometer;

(B) 2 to 10 wt% of polyoxyethylene sorbitan fatty acid ester having an average number of added moles of ethylene oxide (EO) of at least 10 and a hydrophilic-lipophilic balance (HLB) of at least 11, and
(C) 20 to 62 wt% of water.

**2.** An oil-in-water emulsion composition of claim 1, wherein the particles of the emulsion have an average size of 400 nm or less, wherein the average particle size is the volume mean particle size obtained by a laser diffraction method.

**3.** An oil-in-water emulsion composition of claim 1 or 2, wherein component (A-2) is one or more volatile oils selected from hydrocarbon oils and silicone oils having a kinematic viscosity at 25°C of 2 $mm^2/s$ or less, as measured with Ostwald viscometer.

**4.** An oil-in-water emulsion composition of any one of claims 1 to 3, wherein component (B) is one or more selected from polyoxyethylene sorbitan monolaurate, polyoxyethylene sorbitan monostearate, polyoxyethylene sorbitan monooleate and polyoxyethylene sorbitan monopalmitate.

**5.** An oil-in-water emulsion composition of any one of claims 1 to 4, wherein the molar ratio [$R_3SiO_{0.5}$]/[$SiO_2$] between the constituent units in component (A-1) is from 1.1 to 1.4.

**6.** An oil-in-water emulsion composition of any one of claims 1 to 5, wherein component (A-1) is a liquid silicone resin having an absolute viscosity at 36°C of at least 5,000 mPa·s, as measured with a BM-type rotational viscometer.

**7.** An oil-in-water emulsion composition of any one of claims 1 to 6, further comprising (D) 0.1 to 5 wt% of ingredient selected from 1,3-butylene glycol, glycerin, maltitol, sorbitol, 1,2-pentanediol, 1,2-hexanediol, polyethylene glycol and trimethylglycine.

**8.** An oil-in-water emulsion composition of any one of claims 1 to 7, further comprising (E) 0.1 to 5 wt% of water-soluble polymeric compound.

**9.** An oil-in-water emulsion composition of any one of claims 1 to 8, further comprising (F) 0.1 to 10 wt% of synthetic non-crystalline silica.

**10.** An oil-in-water emulsion composition of any one of claims 1 to 9, which is a cosmetic preparation.

## Patentansprüche

**1.** Öl-in-Wasser-Emulsions-Zusammensetzung, die Folgendes umfasst:

(A) 30 bis 70 Gew.-% einer Silikonharz-Zusammensetzung, die ein Gemisch ist, das aus Folgendem besteht:

(A-1) Silikonharz, das [$R_3SiO_{0,5}$]-Einheiten und [$SiO_2$]-Einheiten in einem als [$R_3SiO_{0,5}$]/[$SiO_2$] ausgedrückten Molverhältnis von 0,7 bis 1,4 aufweist, wobei die R jeweils unabhängig eine einwertige organische Gruppe mit 1 bis 10 Kohlenstoffatomen sind, und
(A-2) flüchtiges Öl, das einen Siedepunkt bei Normaldruck von 260 °C oder weniger aufweist,

in einem als (A-1)/(A-2) ausgedrückten Gewichtsverhältnis von 70/30 bis 92/8, und das eine mit einem Ostwald-Viskosimeter gemessene kinematische Viskosität bei 25 °C von 100 bis 4.000 $mm^2/s$ aufweist;
(B) 2 bis 10 Gew.-% Polyoxyethylensorbitanfettsäureester mit einer durchschnittlichen Anzahl an addierten Molen von Ethylenoxid (EO) von zumindest 10 und einem Hydrophil-Lipophil-Gleichgewicht (HLB) von zumindest 11; und

(C) 20 bis 62 Gew.-% Wasser.

2. Öl-in-Wasser-Emulsions-Zusammensetzung nach Anspruch 1, wobei die Teilchen der Emulsion eine mittlere Größe von 400 nm oder weniger aufweisen, wobei die mittlere Teilchengröße die volumenmittlere Teilchengröße ist, die durch ein Laserbeugungsverfahren erhalten wird.

3. Öl-in-Wasser-Emulsions-Zusammensetzung nach Anspruch 1 oder 2, wobei Komponente (A-2) ein oder mehrere flüchtige Öle sind, die aus Kohlenwasserstoffölen und Silikonölen mit einer mit einem Ostwald-Viskosimeter gemessenen kinematischen Viskosität bei 25 °C von 2 $mm^2/s$ oder weniger ausgewählt sind.

4. Öl-in-Wasser-Emulsions-Zusammensetzung nach einem der Ansprüche 1 bis 3, wobei Komponente (B) eine oder mehrere, ausgewählt aus Polyoxyethylensorbitanmonolaurat, Polyoxyethylensorbitanmonostearat, Polyoxyethylensorbitanmonooleat und Polyoxyethylensorbitanmonopalmitat sind.

5. Öl-in-Wasser-Emulsions-Zusammensetzung nach einem der Ansprüche 1 bis 4, wobei das Molverhältnis $[R_3SiO_{0,5}]/[SiO_2]$ zwischen den Bestandseinheiten in Komponente (A-1) 1,1 bis 1,4 beträgt.

6. Öl-in-Wasser-Emulsions-Zusammensetzung nach einem der Ansprüche 1 bis 5, wobei Komponente (A-1) ein flüssiges Silikonharz ist, das eine mit einem Rotationsviskosimeter vom BM-Typ gemessene absolute Viskosität bei 36 °C von zumindest 5.000 mPa·s aufweist.

7. Öl-in-Wasser-Emulsions-Zusammensetzung nach einem der Ansprüche 1 bis 6, die weiters (D) 0,1 bis 5 Gew.-% eines Inhaltsstoffs umfasst, der aus 1,3-Butylenglykol, Glycerin, Maltit, Sorbit, 1,2-Pentandiol, 1,2-Hexandiol, Polyethylenglykol und Trimethylglycin ausgewählt ist.

8. Öl-in-Wasser-Emulsions-Zusammensetzung nach einem der Ansprüche 1 bis 7, die weiters (E) 0,1 bis 5 Gew.-% einer wasserlöslichen Polymerverbindung umfasst.

9. Öl-in-Wasser-Emulsions-Zusammensetzung nach einem der Ansprüche 1 bis 8, die weiters (F) 0,1 bis 10 Gew.-% synthetisches nichtkristallines Siliciumdioxid umfasst.

10. Öl-in-Wasser-Emulsions-Zusammensetzung nach einem der Ansprüche 1 bis 9, die ein kosmetisches Präparat ist.

## Revendications

1. Composition d'émulsion huile dans eau, comprenant :

   (A) de 30 à 70 % en poids d'une composition de résine de silicone qui est un mélange constitué de

      (A-1) une résine de silicone présentant des unités $[R_3SiO_{0,5}]$ et des unités $[SiO_2]$ dans un rapport molaire, exprimé sous la forme $[R_3SiO_{0,5}]/[SiO_2]$, de 0,7 à 1,4, dans laquelle chaque R est indépendamment un groupe organique monovalent de 1 à 10 atomes de carbone, et
      (A-2) une huile volatile présentant un point d'ébullition à pression normale de 260°C ou moins, à un rapport pondéral, exprimé sous la forme (A-1)/(A-2), de 70/30 à 92/8, et présentant une viscosité cinématique à 25°C de 100 à 4 000 $mm^2/s$, telle que mesurée avec un viscosimètre Ostwald ;

   (B) de 2 à 10 % en poids d'ester d'acide gras de polyoxyéthylène sorbitan présentant un nombre moyen de moles d'oxyde d'éthylène (OE) ajoutées d'au moins 10 et un équilibre hydrophile-lipophile (HLB) d'au moins 11, et
   (C) de 20 à 62 % en poids d'eau.

2. Composition d'émulsion huile dans eau selon la revendication 1, dans laquelle les particules de l'émulsion présentent une taille moyenne de 400 nm ou moins, dans laquelle la taille de particule moyenne est la taille de particule moyenne en volume obtenue par un procédé de diffraction laser.

3. Composition d'émulsion huile dans eau selon la revendication 1 ou 2, dans laquelle le composant (A-2) est une ou plusieurs huiles volatiles choisies parmi des huiles hydrocarbonées et des huiles de silicone présentant une viscosité cinématique à 25°C de 2 $mm^2/s$ ou moins, telle que mesurée avec un viscosimètre d'Ostwald.

**4.** Composition d'émulsion huile dans eau selon l'une quelconque des revendications 1 à 3, dans laquelle le composant (B) est un ou plusieurs éléments choisis parmi le monolaurate de polyoxyéthylène sorbitan, le monostéarate de polyoxyéthylène sorbitan, le monooléate de polyoxyéthylène sorbitan et le monopalmitate de polyoxyéthylène sorbitan.

**5.** Composition d'émulsion huile dans eau selon l'une quelconque des revendications 1 à 4, dans laquelle le rapport molaire $[R_3SiO_{05}]/[SiO_2]$ entre les unités constitutives dans le composant (A-1) est de 1,1 à 1,4.

**6.** Composition d'émulsion huile dans eau selon l'une quelconque des revendications 1 à 5, dans laquelle le composant (A-1) est une résine de silicone liquide présentant une viscosité absolue à 36°C d'au moins 5 000 mPa.s, telle que mesurée avec un viscosimètre rotatif de type BM.

**7.** Composition d'émulsion huile dans eau selon l'une quelconque des revendications 1 à 6, comprenant en outre (D) de 0,1 à 5 % en poids d'un ingrédient choisi parmi le 1,3-butylène glycol, la glycérine, le maltitol, le sorbitol, le 1,2-pentanediol, le 1,2-hexanediol, le polyéthylène glycol et la triméthylglycine.

**8.** Composition d'émulsion huile dans eau selon l'une quelconque des revendications 1 à 7, comprenant en outre (E) de 0,1 à 5 % en poids d'un composé polymère hydrosoluble.

**9.** Composition d'émulsion huile dans eau selon l'une quelconque des revendications 1 à 8, comprenant en outre (F) de 0,1 à 10 % en poids de silice non cristalline synthétique.

**10.** Composition d'émulsion huile dans eau selon l'une quelconque des revendications 1 à 9, qui est une préparation cosmétique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2796856 B
- JP 2704730 B
- JP 2017066092 A
- JP 4846423 B
- JP 3220953 B
- JP 5163888 B
- JP 2008138059 A
- FR 2968973 A1